# EUROPEAN PATENT APPLICATION

(11) **EP 3 778 564 A1**
(43) Date of publication of application: **17.02.2021**
(21) Application number: 19777518.2
(22) Date of filing: 18.03.2019
(51) Int. Cl.: C07C 291/04, C07C 231/08, C07C 233/36, C09K 3/00, C07C 231/02

(54) **THICKENING STABILIZER AND SOLVENT COMPOSITION USING SAME FOR PRODUCTION OF ELECTRONIC DEVICE**

(30) Priority: 30.03.2018 JP 2018069615
(71) Applicant: Daicel Corporation, Osaka 530-0011 (JP); Osaka Research Institute of Industrial Science and Technology, Izumi-shi, Osaka 594-1157 (JP)
(72) Inventor: SUZUKI, Youji, Tokyo 108-8230 (JP); KAKEHASHI, Rie, Osaka-shi, Osaka 536-8553 (JP); TOKAI, Naoji, Osaka-shi, Osaka 536-8553 (JP)
(74) Representative: Grünecker Patent- und Rechtsanwälte PartG mbB
(86) International application number: PCT/JP2019/011207
(87) International publication number: WO 2019/188508

(57) **Abstract**

Provided is a compound that thickens a fluid organic material to a desired viscosity and uniformly stabilizes composition thereof. A compound of the present invention is represented by Formula (1): where R¹ represents a monovalent linear aliphatic hydrocarbon group having from 10 to 25 carbons; R² and R³ are the same or different, representing a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different, representing a monovalent aliphatic hydrocarbon group having from 1 to 3 carbon(s) or a hydroxyalkylether group; L¹ to L³ represent an amide bond; in a case where L¹ and L³ are -CONH-, L² is -NHCO-, and in a case where L¹ and L³ are -NHCO-, L² is -CONH-.

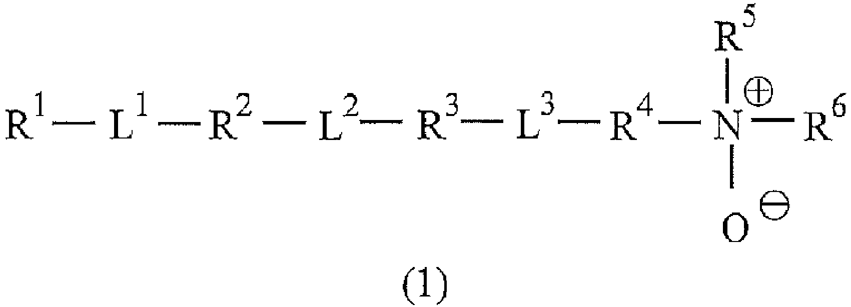

## Description

### Technical Field

The present invention relates to a novel compound for thickening and stabilizing a fluid organic material such as oil, and a solvent composition containing the compound for producing an electronic device. The present application claims priority to the Japanese Patent Application No. 2018-069615 filed on March 30, 2018, the content of which is hereby incorporated herein.

### Background Art

Methods of thickening and stabilizing liquids are industrially very important techniques. For example, mayonnaise and salad dressing, which are emulsions in metastable states, can stably maintain their emulsified state for a long period of time. This is because aqueous components therein are thickened and stabilized. Accordingly, various thickening stabilizers have been developed.

For example, alkyl acrylate copolymers are known as compounds that thicken and stabilize aqueous media.

On the other hand, 12-hydroxystearic acid is known as a thickening stabilizer for fluid organic materials (for example, an organic material having fluidity, such as oily media) (Patent Document 1, etc.). For its gelling action, 12-hydroxystearic acid is mainly used in disposal of edible oils. However, 12-hydroxystearic acid cannot control the degree of gelling; it could only either convert the oil into a solid state completely or leave it in a liquid state. That is, a compound that is capable of thickening a fluid organic material to a desired viscosity has not been found.

### Citation List

### Patent Document

Patent Document 1: JP 01-163111 A

### Summary of Invention

### Technical Problem

Thus, an object of the present invention is to provide a compound that can thicken a fluid organic material to a desired viscosity, and uniformly stabilize the components of the fluid organic material.

Another object of the present invention is to provide a precursor of the compound that can thicken the fluid organic material to a desired viscosity and uniformly stabilize the components of the fluid organic material.

Yet another object of the present invention is to provide a solvent composition including a miscible material of the compound and the fluid organic material, the solvent composition being suitably used for producing an electronic device.

Yet another object of the present invention is to provide a method of producing the solvent composition.

### Solution to Problem

As a result of diligent studies to solve the above problems, the present inventors found that the compound represented by Formula (1) below can, by blending with a fluid organic material to make them miscible, thicken the fluid organic material so as to form a miscible material with its components being uniformly stabilized (preventing sedimentation, local aggregation, or concentration of the composition, and maintaining a uniform state stably), and by adjusting the number of carbons in the compound represented by Formula (1), control the viscosity of the miscible material formed. The present invention was completed based on these findings.

That is, the present invention provides a compound represented by Formula (1) below: 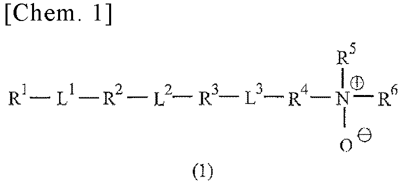
where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group;
L¹ to L³ represent an amide bond; and in a case where L¹ and L³ are - CONH-, L² is -NHCO-, and in a case where L¹ and L³ are -NHCO-, L² is - CONH-.

The present invention also provides a compound represented by Formula (2) below 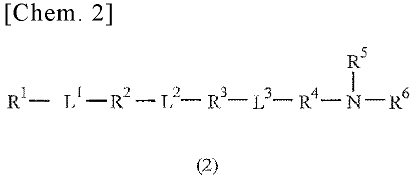 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-.

The present invention also provides a method of producing a compound represented by Formula (2) below, the method including reacting a compound represented by Formula (3) below and a compound represented by Formula (4) below, or reacting a compound represented by Formula (3') below and a compound represented by Formula (4') below to obtain a compound represented by Formula (2); 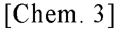 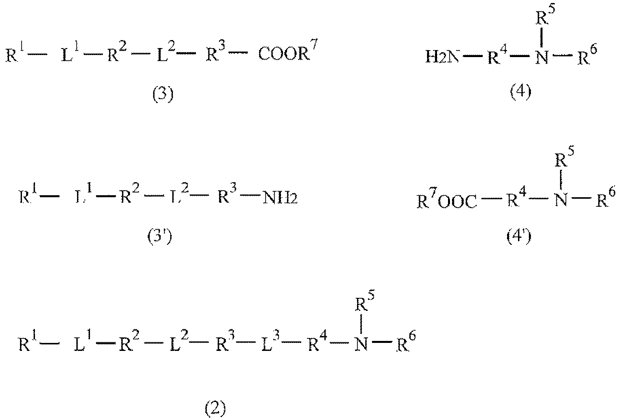 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-; R⁷ represents a hydrogen atom or a alkyl group having from 1 to 3 carbon(s); and in Formula (3), OR⁷, together with a hydrogen atom that constitutes L², may form a ring through a dehydration condensation or a dealcoholization condensation.

The present invention also provides a method of producing a compound represented by Formula (1), the method including oxidizing a compound represented by Formula (2) 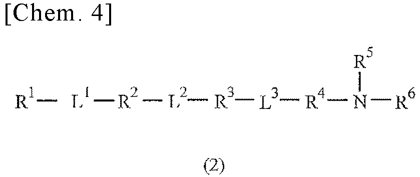 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-;
so as to obtain the compound represented by Formula (1): 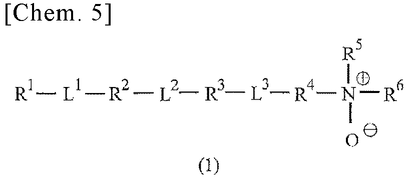 where R¹ to R⁶ and L¹ to L³ are the same as above.

The present invention also provide a solvent composition for producing an electronic device, the solvent composition including a miscible material of the composition represented by Formula (1) above and a fluid organic material.

The present invention also provides a solvent composition for producing an electronic device, in which the fluid organic material is at least one selected from a hydrocarbon oil, an ether, a halogenated hydrocarbon, a petroleum component, an animal and vegetable oil, a silicone oil, an ester, an aromatic carboxylic acid, pyridine, and an alcohol.

The present invention also provides a method of producing a solvent composition for producing an electronic device, the method including making a miscible material from a compound represented by Formula (1) below and a fluid organic material to obtain the solvent composition for producing an electronic device: 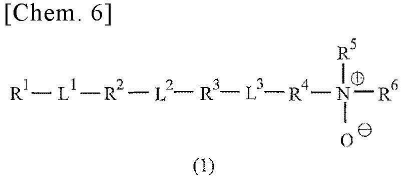 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-.

### Advantageous Effects of Invention

The compound represented by Formula (1) of the present disclosure can, by blending with a fluid organic material to make them miscible, can readily thicken the fluid organic material, and uniformly stabilize the components of the composition containing the fluid organic material. Therefore, the compound can be suitably used as a thickening stabilizer for things such as paint, adhesives, inks, lubricating oils, pharmaceuticals, quasi drugs, and cosmetic products.

In addition, when the compound represented by Formula (2) according to an embodiment of the present invention is oxidized, a compound represented by Formula (1), which is useful as described above, can be produced easily. That is, the compound represented by Formula (2) is extremely useful as a precursor to the compound represented by Formula (1).

Furthermore, the solvent composition including the compound represented by Formula (1) for producing an electronic device according to an embodiment of the present invention has an appropriate viscosity and shear-thinning property. Therefore, the solvent composition is unlikely to cause dripping, and has good coating properties (or discharge properties).

Furthermore, compared to a solvent composition obtained by thickening a fluid organic material with ethyl cellulose, the solvent composition for producing an electronic device according to an embodiment of the present invention can be calcined at a low temperature, allowing to prevent an object to which the present solvent composition is applied from softening and deforming due to a long-time exposure to high temperature. Moreover, a residual amount of ash content after calcining can be significantly reduced, and various problems which may occur due to the presence of the residual ash content (e.g., reduction in electrical properties when used in conductive inks) can be prevented.

### Brief Description of Drawings

FIG. 1 is an ¹H-NMR measurement result of the compound (2-3) obtained in Examples.
FIG. 2 is an ¹H-NMR measurement result of the compound (1-3) obtained in Examples.
FIG. 3 is an ¹H-NMR measurement result of the compound (2-4) obtained in Examples.
FIG. 4 is an ¹H-NMR measurement result of the compound (1-4) obtained in Examples.
FIG. 5 is an ¹H-NMR measurement result of the compound (2-1) obtained in Examples.
FIG. 6 is an ¹H-NMR measurement result of the compound (1-1) obtained in Examples.

### Description of Embodiments

### Compound 1

A compound 1 according to an embodiment of the present invention is represented by Formula (1) below: 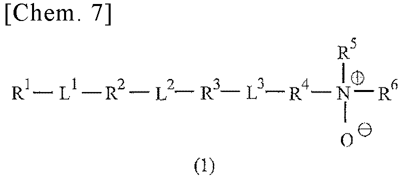 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-.

R¹ is a monovalent linear aliphatic hydrocarbon group having from 10 to 25 carbons, and its examples include a linear alkyl group such as a decyl group, a lauryl group, a myristyl group, a pentadecyl group, a stearyl group, a palmityl group, a nonadecyl group, an eicosyl group, and behenyl group; a linear alkenyl group such as a decenyl group, a pentadecenyl group, an oleyl group, and an eicosenyl group; and a linear alkynyl group such as a pentadecynyl group, an octadecynyl group, and a nonadecyl group.

Among these, for R¹, from the viewpoints of good thickening effect of the fluid organic material and reduction of the residual ash to an extremely small amount upon calcined at a low temperature, R¹ is preferably a monovalent linear aliphatic hydrocarbon group having from 14 to 25 carbons (particularly preferably, an alkyl group having from 14 to 25 carbons), and is particularly preferably a monovalent linear aliphatic hydrocarbon group having from 18 to 21 carbons (particularly preferably an alkyl group having from 18 to 21 carbons).

Examples of the divalent aliphatic hydrocarbon group having 2, 4, 6, or 8 carbons for R² and R³ include an ethylene group, an n-butylene group, an n-hexylene group, and an n-octylene group.

Examples of the divalent alicyclic hydrocarbon group having 6 carbons for R² and R³ include a 1,4-cyclohexylene group, a 1,3-cyclohexylene group, and a 1,2-cyclohexylene group.

Examples of the divalent aromatic hydrocarbon group for R² and R³ include an arylene group having from 6 to 10 carbons, such as a 1,4-phenylene group, a 1,3-phenylene group, and a 1,2-phenylene group.

For R² and R³, among these, from the viewpoints of good thickening effect of the fluid organic material, a divalent aliphatic hydrocarbon group having 2, 4, 6, or 8 carbons is preferable (a linear alkylene group is particularly preferable), a divalent aliphatic hydrocarbon group having 2, 4, or 6 carbons is more preferable (a linear alkylene group is particularly preferable), a divalent aliphatic hydrocarbon group having 2 or 4 carbons is even more preferable (a linear alkylene group is particularly preferable), and a divalent aliphatic hydrocarbon group having 2 carbons is the most preferable (a linear alkylene group is particularly preferable).

R⁴ represents a divalent aliphatic hydrocarbon group having from 1 to 8 carbon(s). Among these, from the viewpoints of good thickening effect of the fluid organic material, a linear or branched alkylene group is preferable, and a linear alkylene group is particularly preferable.

Furthermore, R⁴ represents a divalent aliphatic hydrocarbon group having from 1 to 8 carbon(s). Among these, from the viewpoints of good thickening effect of the fluid organic material, a divalent aliphatic hydrocarbon group having from 1 to 7 carbon(s) is more preferable, a divalent aliphatic hydrocarbon group having from 3 to 7 carbons is particularly preferable, a divalent aliphatic hydrocarbon group having from 3 to 6 carbons is the most preferable, and a divalent aliphatic hydrocarbon group having from 3 to 5 carbons is even particularly preferable.

Therefore, for R⁴, it is preferably a linear or branched alkylene group having from 1 to 8 carbon(s), more preferably a linear alkylene group having from 1 to 7 carbon(s), particularly preferably a linear alkylene group having from 3 to 7 carbons, most preferably a linear alkylene group having from 3 to 6 carbons, and particularly preferably a linear alkylene group having from 3 to 5 carbons.

Examples of the monovalent aliphatic hydrocarbon group having from 1 to 3 carbon(s) for R⁵ and R⁶ include a linear or branched alkyl group having from 1 to 3 carbons, such as a methyl group, an ethyl group, a propyl group, and an isopropyl group; a linear or branched alkenyl group having from 2 to 3 carbons, such as a vinyl group, a 1-methylvinyl group and 2-propenyl group; and a linear or branched alkynyl group having from 2 to 3 carbons, such as an ethynyl group and a propynyl group.

Examples of the hydroxyalkylether group for R⁵ and R⁶ include a mono- or di- (hydroxy)C₁₋₃ alkylether group, such as a 2-hydroxyethoxy group, a 2-hydroxypropoxy group, and a 2,3-dihydroxypropoxy group.

Among these, R⁵ and R⁶ may be the same or different, and are preferably a monovalent aliphatic hydrocarbon group having from 1 to 3 carbon(s), and more preferably a linear or branched alkyl group having from 1 to 3 carbon(s), particularly preferably a linear alkyl group having from 1 to 3 carbon(s), and even particularly preferably a methyl group.

Among the compounds represented by Formula (1), the compounds represented by Formula (1-1) to (1-9) below are particularly preferred from the viewpoint of excellent solubility a fluid organic material. Additionally, in a case where the fluid organic material is transparent, the compounds can thicken and stabilize the fluid organic material while maintaining its transparency.

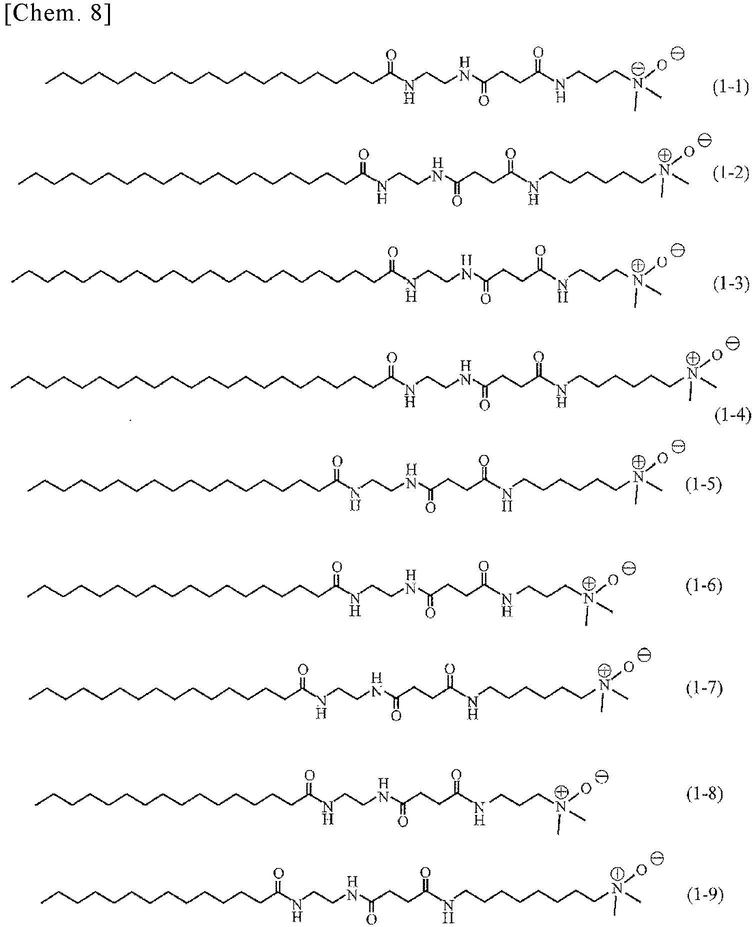

### Compound 2

Compound 2 according to an embodiment of the present invention is a compound represented by Formula (2) below. Compound 2 is useful as a precursor for Compound 1 described above. 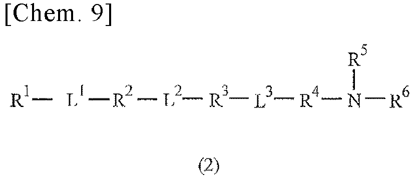 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) is a hydroxyalkylether group; L¹ to L³ represent an amide bond; and in a case where L¹ and L³ are -CONH-, L² is -NHCO-, and in a case where L¹ and L³ are - NHCO-, L² is -CONH-.

R¹ to R⁶ and L¹ to L³ in Formula (2) above are the same as described above.

For the compound represented by Formula (2), the compounds represented by Formulas (2-1) to (2-9) below are particularly preferable.

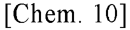

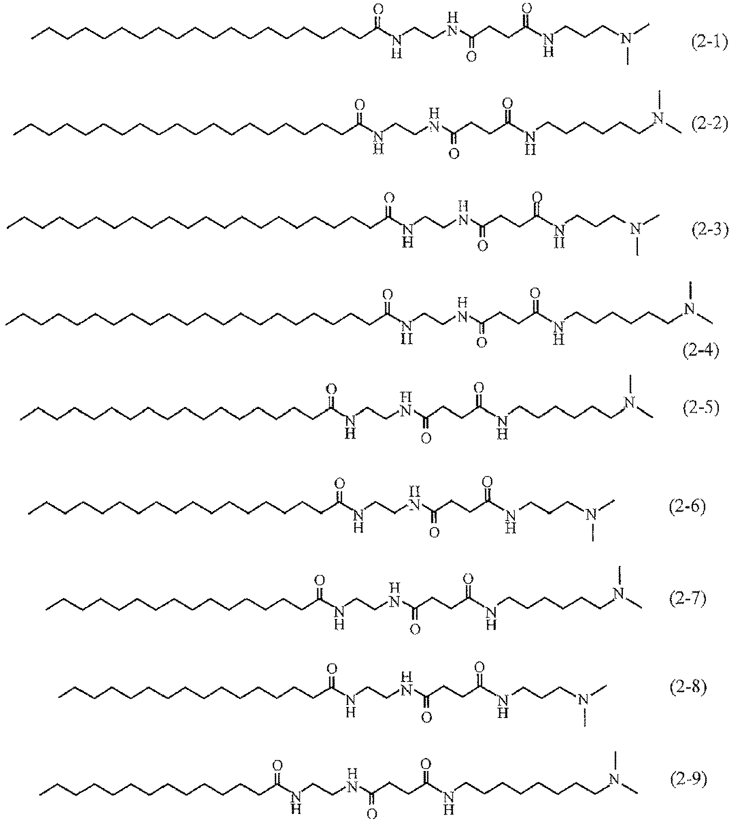

### Method of Producing Compound 1 and Compound 2

Compound 2 is formed by reacting a compound represented by Formula (3) below (hereinafter, also referred to as "Compound 3") and a compound represented by Formula (4) below (hereinafter, also referred to as "Compound 4"); or by reacting a compound represented by Formula (3') below (hereinafter, also referred to as "Compound 3''') and a compound represented by Formula (4') below (hereinafter, also referred to as "Compound 4'''). 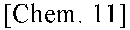 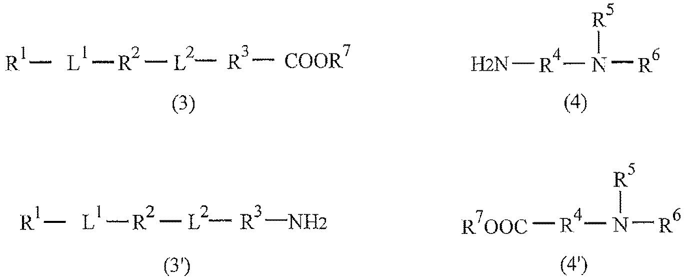 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-; R⁷ represents a hydrogen atom or a alkyl group having from 1 to 3 carbon(s); and in Formula (3), OR⁷, together with a hydrogen atom that constitutes L², may form a ring through a dehydration condensation or a dealcoholization condensation.

R¹ to R⁶ and L¹ to L² in Formula above are the same as described above.

Examples of the alkyl group having from 1 to 3 carbon(s) in R⁷ in formulas (3) and (4') include a methyl group, an ethyl group, a propyl group, and an isopropyl group.

Examples of the ring formed through a dehydration condensation or a dealcoholization condensation of OR⁷ in Formula (3) with the hydrogen atom that constitutes L² include a pyrrolidine-2,5-dione ring, and a piperidine-2,6-dione ring.

The amount of Compound 4 that is used may be not less than 1 mol per 1 mol of Compound 3, and an excessive amount of Compound 4 can be used.

The amount of Compound 4' that is used may be not less than 1 mol per 1 mol of Compound 3', and an excessive amount of Compound 4' may be used.

A reaction of Compound 3 and Compound 4, or Compound 3' and Compound 4' can be carried out, for example, by stirring at a temperature from 100 to 120°C for 10 to 20 hours.

The reaction atmosphere of the reaction above is not particularly limited as long as it does not inhibit the reaction. For example, any of an air atmosphere, a nitrogen atmosphere, and an argon atmosphere may be used. In addition, the reaction can be performed by any method, such as a batch method, a semi-batch method, and a continuous method.

After the completion of the reaction, the resulting reaction product can be separated and purified by a separation method, such as filtration, concentration, distillation, extraction, crystallization, adsorption, recrystallization, and column chromatography; and a separation method in combination thereof.

Furthermore, Compound 1 can be produced by forming Compound 2 using the method described above, and then oxidizing Compound 2 formed.

For the oxidizing agent used in the oxidation of Compound 2 formed in the method described above, hydrogen peroxide can be used, for example. For the hydrogen peroxide above, pure hydrogen peroxide may be used, but from the viewpoint of handling, the hydrogen peroxide is typically diluted with a suitable solvent (e.g., water) (e.g., from 5 to 70 wt% hydrogen peroxide water) and used. The amount of hydrogen peroxide that is used is, for example, approximately from 0.1 to 10 mol per 1 mol of Compound 2.

The oxidation reaction can be carried out, for example, by stirring at a temperature from 30 to 70°C for 3 to 20 hours.

The oxidation reaction of Compound 2 is carried out in the presence or absence of a solvent. Examples of the solvent include an alcohol-based solvent such as methanol, ethanol, 2-propanol, and butanol; an ether-based solvent such as diethyl ether, diisopropyl ether, dibutyl ether, tetrahydrofuran, dioxane, dioxolane, 1,2-dimethoxyethane, and cyclopentyl methyl ether; an ester-based solvent such as butyl acetate and ethyl acetate; a hydrocarbon-based solvent such as pentane, hexane, heptane, and octane; and a nitrile-based solvent such as acetonitrile and benzonitrile. One of these alkali catalysts can be used alone, or two or more of these catalysts can be used in combination.

After the completion of the reaction, the resulting reaction product can be separated and purified by a separation method, such as filtration, concentration, distillation, extraction, crystallization, adsorption, recrystallization, and column chromatography; and a separation method in combination thereof.

As Compound (3) that serves as a raw material for the reaction above, a compound represented by Formula (3-1) below can be produced by, for example, a method described below. Note that R¹, R², R³, and R⁷ in the formula below are the same as those described above. Also, R⁷ in Formula (3a) and two R⁷ s in Formula (3d) may be the same or different from each other. Furthermore, the compound represented by Formula (3d) may be in an anhydride form as the result of a dehydration condensation of the two COOR⁷ in the formula.

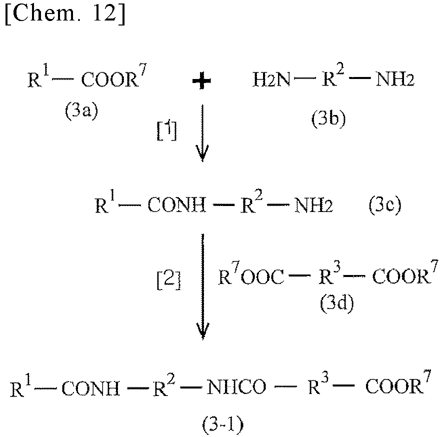

As Compound 3' that serves as a raw material for the reaction above, a compound represented by Formula (3'-1) below can be produced by, for example, a method described below. Note that R¹, R², R³, and R⁷ in the formula below are the same as those described above. Two R⁷s in Formula (3b') may be the same or different. Furthermore, the compound represented by Formula (3b') may be in an anhydride form as the result of a dehydration condensation of the two COOR⁷ in the formula.

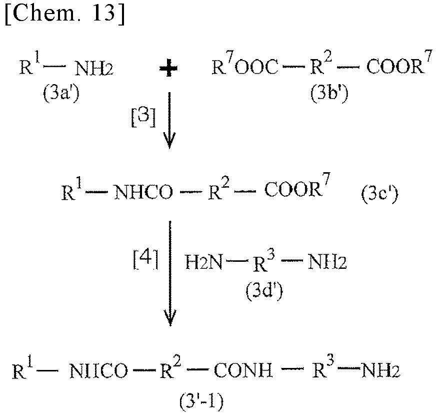

In the process [1], the compound represented by Formula (3a) and the compound represented by formula (3b) are reacted and the compound represented by formula (3c) is formed. The amount of the compound represented by formula (3b) that is used may be not less than 1 mol per 1 mol of the compound represented by formula (3a), and an excessive amount of the compound can be used. The reaction temperature of the reaction is, for example, from 80 to 150°C, and the duration of the reaction is, for example, approximately from 1 to 24 hours.

In the process [2], the compound represented by formula (3c) and the compound represented by formula (3d) are reacted and the compound represented by formula (3-1) is formed. The amount of the compound represented by formula (3d) that is used may be not less than 1 mol, and preferably from 1 to 3 mol, per 1 mol of the compound represented by formula (3c). The reaction temperature of the reaction is, for example, from 80 to 150°C, and the duration of the reaction is, for example, approximately from 0.5 to 10 hours. As the reaction proceeds, water is produced. Therefore, the reaction is preferably carried out while water is being removed using a dehydrating agent (for example, acetic anhydride) thereby promoting the progress of the reaction.

The reaction in [2] is preferably carried out in the presence of a solvent. Examples of the solvent include pentafluorophenol, N,N-dimethylformamide, dimethylacetamide, and o-dichlorobenzene. One of these alkali catalysts can be used alone, or two or more of these catalysts can be used in combination.

As necessary, the reaction in [2] may also be carried out in the presence of a base such as triethylamine, pyridine, and 4-dimethylaminopyridine.

In the process [3], the compound represented by Formula (3a') and the compound represented by formula (3b') are reacted and the compound represented by formula (3c') is formed. The reaction in [3] may be carried out under the condition according to the condition for the reaction in [2] above.

In the process [4], the compound represented by formula (3c') and the compound represented by formula (3d') are reacted and the compound represented by formula (3'-1) is formed. The reaction in [4] may be carried out under the condition according to the condition for the reaction in [1] above.

After the completion of the reaction in each of the processes, the resulting reaction product can be separated and purified by a separation method, such as filtration, concentration, distillation, extraction, crystallization, adsorption, recrystallization, and column chromatography; and a separation method in combination thereof.

### Solvent Composition for Producing Electronic Device

The solvent composition for producing an electronic device according to an embodiment of the present invention is a composition including a miscible material of Compound 1 and a fluid organic material, and a composition in which the fluid organic material is thickened and the components are uniformly stabilized by Compound 1.

The fluid organic material serving as a raw material is an organic material having a viscosity (η) at 25°C and a shear rate of 1 s⁻¹ of, for example, less than 0.5 Pa·s. Examples of the fluid organic material include a hydrocarbon oil, such as hexane, cyclohexane, isododecane, benzene, toluene, poly-α-olefins, and liquid paraffin; ethers, such as tetrahydrofuran; a halogenated hydrocarbon, such as carbon tetrachloride and chlorobenzene; a petroleum component, such as kerosene, gasoline, gas oil, and heavy oil; an animal and vegetable oil, such as a sunflower oil, an olive oil, a soy bean oil, a corn oil, a castor oil, beef fat, a jojoba oil, and squalanes; a silicone oil, such as dimethyl polysiloxanes and methylphenylpolysiloxane; esters, such as octyldodecyl oleate, cetyl octanoate, cetyl ethylhexanoate, glyceryl triisooctanoate, and neopentyl glycol diisooctanoate; an aromatic carboxylic acid; pyridine; alcohols, such as α-terpineol, dihydroterpineol, ethylene glycol, 1,2-propane diol, 1,3-propanediol, 1,2-butanediol, 1,3-butanediol, and 1,4-butanediol. One of these alkali catalysts can be used alone, or two or more of these catalysts can be used in combination.

The solvent composition for producing an electronic device can be produced via a process of blending Compound 1 and the fluid organic material to make them miscible. More specifically, the solvent composition for producing an electronic device can be produced by mixing and warming the total amount of the fluid organic material and Compound 1 to make them miscible, and then cooling thereafter. The solvent composition for producing an electronic device can be also produced by a method of mixing Compound 1 in a portion of the fluid organic material, warming the mixture to make a miscible material, and then cooling the material to produce a solvent composition for producing an electronic device, and then mixing this composition in the remaining fluid organic material.

The temperature during the making a miscible material is selected as appropriate for the types of Compound 1 and the fluid organic material used, and is not particularly limited as long as it is a temperature at which Compound 1 and the fluid organic material are miscible. The temperature is preferably not higher than 100°C, and in a case where the boiling point of the fluid organic material is 100°C or lower, the temperature is preferably around the boiling point.

The cooling after the making the miscible material may be performed in any manner, as long as it is capable of cooling to a room temperature (e.g. 25°C) or lower, and it may be performed by a gradual cooling at room temperature or a rapid cooling, such as ice cooling.

Compound 1 may be used in an amount, for example, from 0.1 to 100 parts by weight, preferably from 0.5 to 80 parts by weight, particularly preferably from 1 to 60 parts by weight, and most preferably from 1 to 30 parts by weight, relative to 1000 parts by weight of the fluid organic material, although the amount depends on the type of the fluid organic material. Compound 1 is used in the range above, and thus the compound (or the miscible material) is formed, in which the fluid organic material is thickened and the components are uniformly stabilized.

The solvent composition for producing an electronic device according to an embodiment of the present invention may contain other components in a range that does not impair the effects of the present invention in addition to the miscible material of Compound 1 and the fluid organic material. The amount of the miscible material contained therein with respect to the total of the solvent composition for producing an electronic device (or the total amount of Compound 1 and the fluid organic material) may be, for example, not less than 30 wt.%, preferably not less than 50 wt.%, particularly preferably not less than 60 wt.%, the most preferably not less than 70 wt.%, and even particularly preferably not less than 90 wt.%. Note that the upper limit is 100 wt.%. That is, the solvent composition for producing an electronic device according to an embodiment of the present invention contains substantially no other components, and may only consist of the miscible material of Compound 1 and the fluid organic material. The other components can be appropriately adjusted according to the application.

And the viscosity (η) [at 25°C, shear rate 0.3 s⁻¹] of the solvent composition for producing an electronic device according to an embodiment of the present invention, measured by a rheometer, is preferably in a range of 10 Pa·s or greater (e.g. from 10 to 100 Pa·s), and such a viscosity can preferably help suppressing dripping or flowing of the composition after coating, thereby improving the coating accuracy.

Furthermore, the viscosity (η) [at 25°C, shear rate 0.1 s⁻¹] of the solvent composition for producing an electronic device according to an embodiment of the present invention, measured by a rheometer, is preferably in a range of 10 Pa·s or greater (e.g., from 10 to 100 Pa·s), and such a viscosity can preferably help suppressing dripping or flowing of the composition after coating, thereby improving the coating accuracy.

The solvent composition for producing an electronic device according to an embodiment of the present invention has a shear-thinning property, and the viscosity ratio [viscosity measured by the rheometer at 25°C and shear rate of 1 s⁻¹/viscosity measured by the rheometer at a shear rate of 10 s⁻¹] is, for example, greater than 1.5, preferably 2 or greater, and particularly preferably 3 or greater. And the upper limit is, for example, 10 and preferably 8. Therefore, the viscosity can be reduced during coating, and, for example, excellent discharge properties can be achieved when coating the solvent composition using a printing machine or the like. Moreover, the rapid increase in the viscosity after coating can help suppressing dripping of the composition that has been coated, thereby improving the coating accuracy.

The solvent composition for producing an electronic device according to an embodiment of the present invention has moderate viscosity and a shear-thinning property as described above, there is no need to add a binder resin (for example, a polymeric compound having a molecular weight of not less than 10000, such as ethyl cellulose resin, alkyl cellulose resin, polyvinyl acetal resin, and acrylic resin). Even in a case where the binder resin is added, the added amount is, for example, not greater than 10 wt.% of the total amount of the composition (100 wt.%), and is preferably not greater than 5 wt.%. When the added amount of the binder resin exceeds the range described above, the residual content of the ash from calcining the binder resin may increase and the various problems caused by the residual ash content, such as reduction in electrical properties when used in conductive inks, may not be suppressed very easily.

In addition, the solvent composition for producing an electronic device according to an embodiment of the present invention has excellent thermal degradability and its molecular weight can be easily reduced. Therefore, the solvent composition for producing an electronic device according to an embodiment of the present invention can be calcined at a lower temperature (for example, from 100 to 350°C, preferably from 150 to 300°C, and particularly preferably from 150 to 250°C) compared to a solvent composition having a viscosity imparted by a binder resin such as ethyl cellulose, and the softening and deformation of the object to be coated during calcining can be prevented.

The solvent composition for producing an electronic device according to an embodiment of the present invention has the characteristics described above in combination, and is useful as a solvent for an ink for forming a wiring and/or an electrode in production of an electronic device such as a capacitor, an inductor, a varistor, a thermistor, a speaker, an actuator, an antenna, a solid oxide fuel cell (SOFC). Furthermore, solvent composition for producing an electronic device according to an embodiment of the present invention is particularly useful as a solvent for an adhesive in the production of the electronic device.

### Examples

Hereinafter, the present invention will be described more specifically with reference to examples, but the present invention is not limited by these examples.

### Example 1: Preparation of Compound 1-3

Methyl docosanoate (20.0 g, 56.4 mmol) and ethylenediamine (16.9 g, 281 mmol) were stirred at 110°C for 18 hours and the reaction product was washed with methanol and then filtered. The filtrate was removed by solvent distillation, and the obtained residue was purified by recrystallization using hexane. The white crystal of N-docosanoylethylenediamine was obtained (yield: 65%, 14.0 g, 36.7 mmol).

In a solution of N-dimethylformamide (40 ml), N-docosanoylethylenediamine (12.0 g, 31.4 mmol) and triethylamine (6.35 g, 62.8 mmol) were added to succinic anhydride (3.45 g, 34.5 mmol) over 10 minutes and stirred at 100°C for 15 minutes. After succinic anhydride was dissolved, acetic anhydride (4.81 g, 47.1 mmol) was added dropwise over 10 minutes to the reaction crude solution and stirred at 100°C for 1 hour. The reaction mixture was poured into water (200 ml), the precipitate was filtered and washed with water. The precipitate was purified by recrystallization using 2-propanol. The white crystalline powders of N-docosanoylethylsuccineimide were obtained (yield: 92%, 13.4 g, 28.9 mmol).

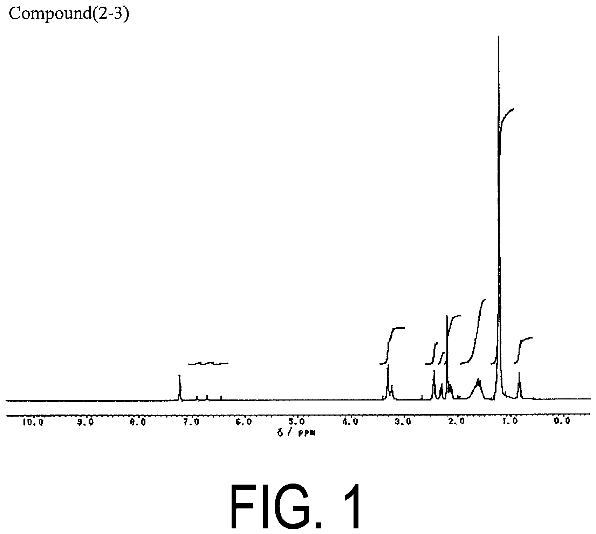

N-docosanoylethylsuccineimide (4.00 g, 8.60 mmol) and N,N-dimethyl - 1,3-propanediamine (2.63 g, 25.8 mmol) were stirred at 120°C for 18 hours. The reaction mixture was poured into methanol and the precipitate was filtered and washed with methanol. The resulting solid was purified by recrystallization using acetone and methanol. The white crystalline powders of N-(docosanoylaminoethylaminosuccinamoylaminopropyl)-N,N-dimethylamine (compound 2-3) represented by Formula (2-3) below were obtained (yield: 94%, 4.58 g, 8.08 mmol). The ¹H-NMR (CDCl₃) measurement result of the resulting compound is shown in FIG. 1.

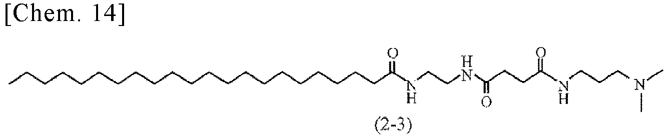

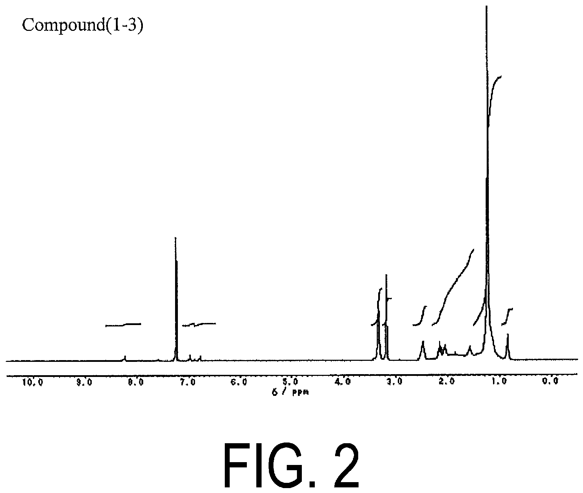

N-(docosanoylaminoethylaminosuccinamoylaminopropyl)-N,N-dimethylamine (4.00 g, 7.06 mmol), 35% hydrogen peroxide (2.06 ml) and 2-propanol (10 ml) were stirred at 60°C for 5 hours. Palladium carbon (approximately 10 mg) was added to the reaction solution, and the reaction solution was further stirred for 18 hours at room temperature. The reaction solution was filtered and the solvent was distilled off, and then purified by column chromatography (silica gel, 2-propanol/methanol). The white solid of N-(docosanoylaminoethylaminosuccinamoylaminopropyl)-N,N-dimethylamine oxide (compound 1-3) represented by Formula (1-3) below was obtained (yield: 69%, 2.84 g, 4.87 mmol). The ¹H-NMR (CDCl₃) measurement result of the resulting compound is shown in FIG. 2.

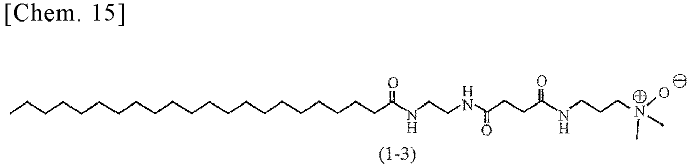

### Example 2: Preparation of Compound 1-4

N-docosanoylethylsuccineimide was obtained by the same method as in Example 1.

The resulting N-docosanoylethylsuccineimide (8.00 g, 17.2 mmol) and hexamethylene diamine (10.0 g, 86.1 mmol) were stirred at 120°C for 18 hours. The reaction mixture was poured into methanol and the precipitate was filtered and washed with methanol. The resulting solid was purified by recrystallization using acetonitrile, methanol. The white crystalline powders of N-(docosanoylaminoethyl)aminosuccinamoylaminohexylamine were obtained (yield: 69%, 6.91 g, 11.9 mmol).

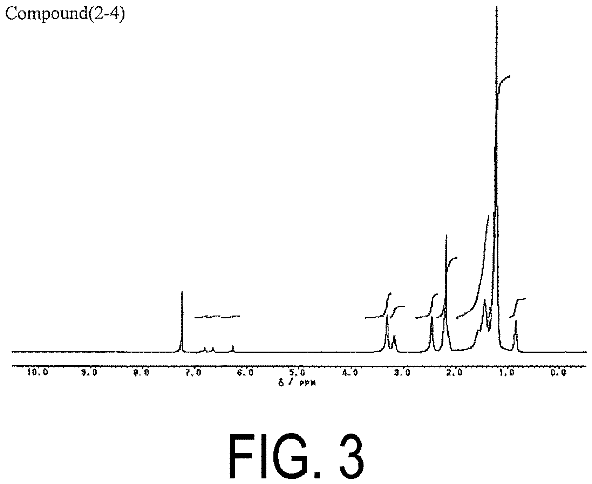

N-(docosanoylaminoethyl)aminosuccinamoylaminohexylamine (3.25 g, 5.59 mmol), 37% aqueous formaldehyde (2.73 ml) and formic acid (1.55 g, 33.7 mmol) were dissolved in 2-propanol (15 ml) and stirred at 100°C for 4 hours. The reaction mixture was poured into 1 M aqueous sodium hydroxide solution (20 ml) and the crystals were filtered. The resulting crystals were recrystallized with methanol and acetone, and the white solid of N-(docosanoylaminoethylaminosuccinamoylaminohexyl)-N, N-dimethylamine (compound 2-4) represented by Formula (2-4) below (yield: 89%, 3.03 g, 4.98 mmol) was obtained. The ¹H-NMR (CDCl₃) measurement results of the resulting compound are shown in FIG. 3.

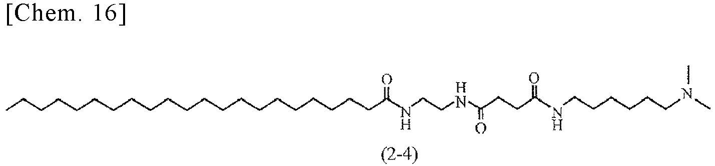

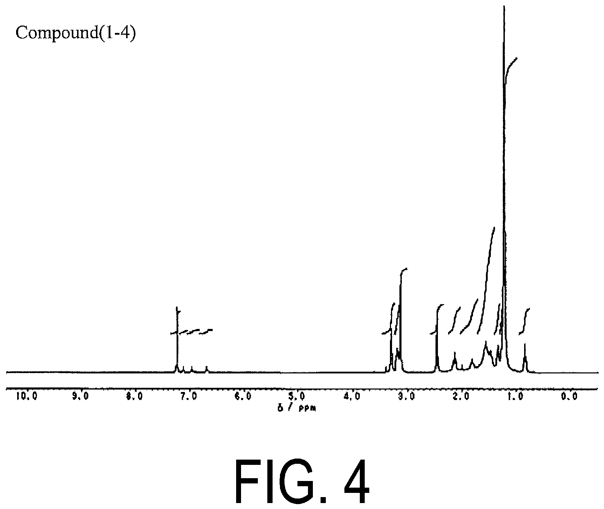

N-(docosanoylaminoethylaminosuccinamoylaminohexyl)-N,N-dimethylamine (2.80 g, 4.60 mmol), 35% hydrogen peroxide (1.30 ml) and, 2-propanol (10 ml) were stirred at 60°C for 5 hours. Palladium carbon (approximately 10 mg) was added to the reaction solution, and the reaction solution was further stirred for 18 hours at room temperature. The reaction solution was filtered and the solvent was distilled off, and then purified by column chromatography (silica gel, 2-propanol/methanol). The white solid of N-(docosanoylaminoethylaminosuccinamoylaminohexyl)-N, N-dimethylamine oxide (compound 1-4) represented by Formula (1-4) below (yield: 74%, 2.13 g, 3.40 mmol) was obtained. The ¹H-NMR (CDCl₃) measurement result of the resulting compound is shown in FIG. 4.

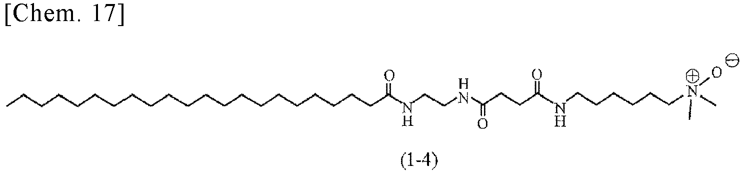

### Example 3: Preparation of Compound 1-1

Methyl eicosanoate (18.0 g, 55.1 mmol) and ethylenediamine (16.5 g, 276 mmol) were stirred at 110°C for 18 hours and the reaction product was washed with methanol and then filtered. The filtrate was removed by solvent distillation, and the obtained residue was purified by recrystallization using hexane. The white crystal of N-eicosanoylethylenediamine was obtained (yield: 68%, 13.3 g, 37.5 mmol).

In a solution of N-dimethylformamide (30 ml), N-eicosanoylethylenediamine (10.0 g, 28.2 mmol) and triethylamine (5.71 g, 56.4 mmol) were added to succinic anhydride (3.10 g, 31.0 mmol) over 10 minutes and stirred at 100°C for 15 minutes. After succinic anhydride was dissolved, acetic anhydride (4.32 g, 42.3 mmol) was added dropwise over 10 minutes to the reaction crude solution and stirred at 100°C for 1 hour. The reaction mixture was poured into water (150 ml), the precipitate was filtered and washed with water. The precipitate was purified by recrystallization using 2-propanol. The white crystalline powders of N-eicosanoylaminoethylsuccineimide were obtained at 91% yield (11.2 g, 25.7 mmol).

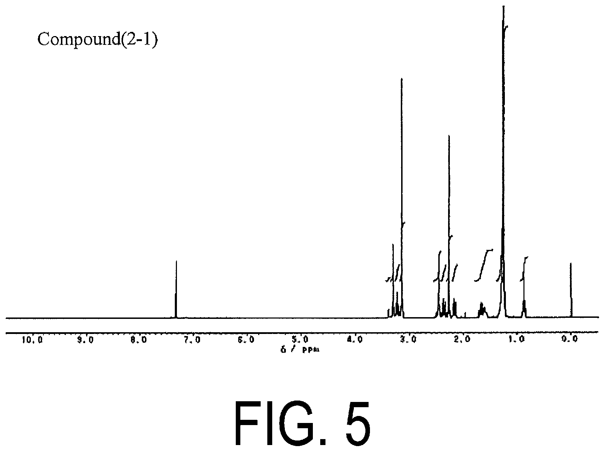

N-eicosanoylaminoethylsuccineimide (4.00 g, 9.16 mmol) and N,N-dimethyl-1,3-propanediamine (2.81 g, 27.5 mmol) were stirred at 120°C for 18 hours. The reaction mixture was poured into methanol and the precipitate was filtered and washed with methanol. The resulting solid was purified by recrystallization using acetone and methanol. The white crystalline powders of N-(eicosanoylaminoethylaminosuccinamoylaminopropyl)-N,N-dimethylamine (compound 2-1) represented by Formula (2-1) below were obtained (yield: 91%, 4.49 g, 8.34 mmol). The ¹H-NMR (CDCl₃) measurement result of the resulting compound is shown in FIG. 5.

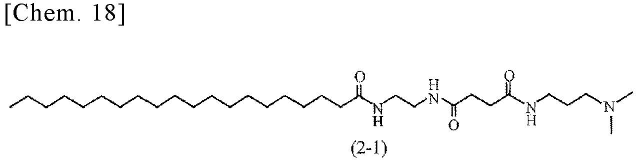

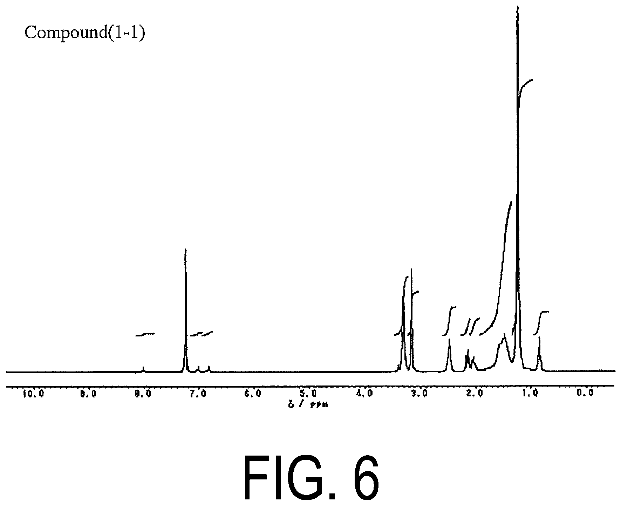

N-(eicosanoylaminoethylaminosuccinamoylaminopropyl)-N,N-dimethylamine (4.00 g, 7.42 mmol), 35% hydrogen peroxide (2.16 ml) and 2-propanol (10 ml) were stirred at 60°C for 5 hours. Palladium carbon (approximately 10 mg) was added to the reaction solution, and the reaction solution was further stirred for 18 hours at room temperature. The reaction solution was filtered and the solvent was distilled off, and then purified by column chromatography (silica gel, 2-propanol/methanol). The white solid of N-(eicosanoylaminoethylaminosuccinamoylaminopropyl)-N,N-dimethylamine oxide (compound 1-1) represented by Formula (1-1) below was obtained (yield: 58%, 2.39 g, 4.30 mmol). The ¹H-NMR (CDCl₃) measurement result of the resulting compound is shown in FIG. 6.

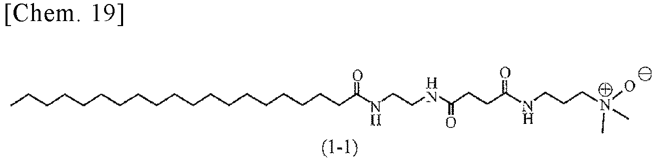

### Example 4: Preparation of Compound 1-5

A compound represented by Formula (2-5) below was obtained in the same manner as in Example 2 with the exception that methyl octadecanoate was used instead of methyl docosanoate. And the compound represented by Formula (1-5) below was obtained.

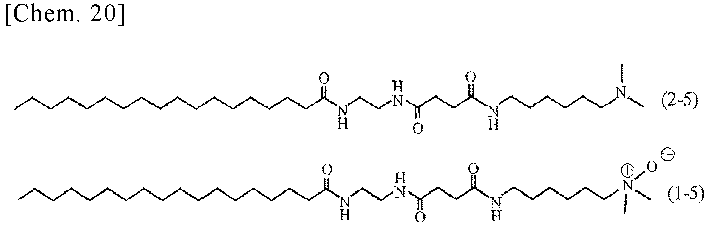

### Example 5: Preparation of Compound 1-6

A compound represented by Formula (2-6) below was obtained in the same manner as in Example 1 with the exception that methyl octadecanoate was used instead of methyl docosanoate. And the compound represented by Formula (1-6) below was obtained.

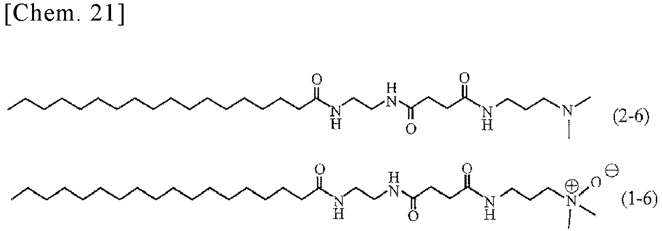

### Example 6: Preparation of Compound 1-7

A compound represented by Formula (2-7) below was obtained in the same manner as in Example 2 with the exception that methyl palmitate was used in place of methyl docosanoate. And the compound represented by Formula (1-7) below was obtained.

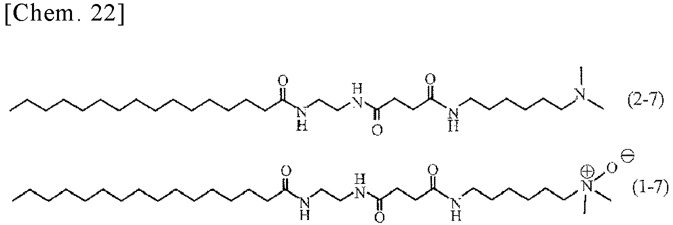

### Example 7: Production of Compound 1-8

A compound represented by Formula (2-8) below was obtained in the same manner as in Example 1 with the exception that methyl palmitate was used in place of methyl docosanoate. And the compound represented by Formula (1-8) below was obtained.

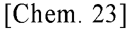

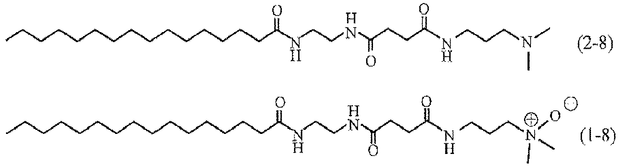

### Example 8: Preparation of Compound 1-9

A compound represented by formula (2-9) below was obtained in the same manner as in Example 2 with the exception that methyl myristate was used instead of methyl docosanoate. And the compound represented by Formula (1-9) below was obtained.

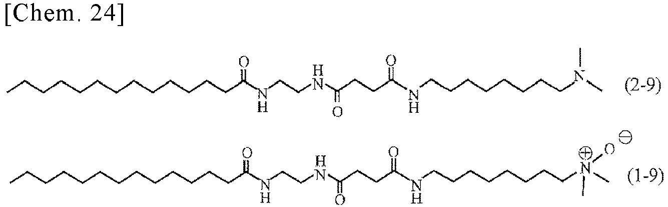

### Examples 9 to 16 and Comparative Examples 1 to 2, Production of Solvent Composition

1 cm³ of the various fluid organic materials listed in Table 1 (1,3-butanediol (1,3-BG), α-terpineol (TPO)) were each sampled in each test tube; 10 mg of each of the compounds obtained in Example 1 to 8 above were added as a thickening stabilizer, mixed, and heated and stirred at 100°C to make a miscible material of the fluid organic material and the thickening stabilizer. The miscible material was cooled to 25°C, and a solvent composition was obtained. Note that in the comparative examples, ethyl cellulose (EC: trade name "ETHOCEL STD200", available from Nissin Kasei Co., Ltd.) was used as the thickening stabilizer. The fluid organic material and the thickening stabilizer were heated and dissolved for 24 hours at a liquid temperature of 80°C and cooled to 25°C to prepare a solvent composition.

The viscosity of the solvent composition was determined as follows. The measurement was performed using a viscosity/visco-elasticity measuring apparatus (rheometer) (trade name "RheoStress 600" available from HAAKE) equipped with a cone-plate sensor (a cone-plate sensor with a diameter of 60 mm with a cone angle of 1°, and cone-plate sensors with a diameter of 35 mm with a cone angle of 1°, 2°, and 4° were used) and a Peltier temperature controller. The viscosity was measured in a steady flow viscosity measurement mode at 25°C and different shear rates varied in a log scale from 0.1 to 100 s⁻¹. Thus, the thickening effect (shear-thinning property and thickening property) were evaluated.

### Shear-thinning property evaluation

For the solvent compositions obtained, [(viscosity at a shear rate of 1 s⁻¹) / (viscosity at a shear rate of 10 s⁻¹] was evaluated for shear-thinning property according to the following criteria.
1: 1.5 or less
2: Greater than 1.5 and 3.0 or less
3: Greater than 3.0 and 4.5 or less
4: Greater than 4.5

### Thickening Property Evaluation

For the solvent composition obtained, the viscosity at a shear rate of 0.1 s⁻¹ was evaluated for thickening property according to the following criteria.
1: 5 Pa·s or less
2: Greater than 5 Pa·s and 10 Pa·s or less
3: Greater than 10 Pa·s and 50 Pa·s or less
4: Greater than 50 Pa·s

In addition, the residual ash content of the solvent compositions obtained in the Examples and Comparative Examples at 250°C was measured by the following method.

Using a TG-DTA, 20 mg of each of the solvent compositions was heated from 20°C to 400°C at 10°C/minute, and the residual ash amount at 250°C was measured. The proportion of residual ash amount with respect to the total amount of the solvent composition (= residual ash content) was calculated.

The results are summarized and shown in the table below.

**[Table 1]**

| | Thickening stabilizer | | | | | | | | | Fluid organic materials | | Shear-thinning property | Thickening property | Residual ash content (wt%) |
|---|---|---|---|---|---|---|---|---|---|---|---|---|---|---|
| | Compound 1-3 | Compound 1-4 | Compound 1-1 | Compound 1-5 | Compound 1-6 | Compound 1-7 | Compound 1-8 | Compound 1-9 | EC | 1,3-BG | TPO | | | |
| Example 9 | 1 | - | - | - | - | - | - | - | - | 99 | - | 4 | 4 | 0.8 |
| Example 10 | - | 1 | - | - | - | - | - | - | - | 99 | - | 3 | 4 | 0.6 |
| Example 11 | - | - | 1 | - | - | - | - | - | - | 99 | - | 4 | 4 | 0.9 |
| Example 12 | - | - | - | 1 | - | - | - | - | - | 99 | - | 3 | 2 | 0.7 |
| Example 13 | - | - | - | - | 1 | - | - | - | - | 99 | - | 4 | 3 | 0.8 |
| Example 14 | - | - | - | - | - | 1 | - | - | - | 99 | - | 2 | 1 | 0.9 |
| Example 15 | - | - | - | - | - | - | 1 | - | - | 99 | - | 3 | 3 | 1.6 |
| Example 16 | - | - | - | - | - | - | - | 1.5 | - | 98.5 | - | 4 | 3 | 1.6 |
| Comparative Example 1 | - | - | - | - | - | - | - | - | 5 | 95 | - | 1 | 2 | 4.9 |
| Comparative Example 2 | - | - | - | - | - | - | - | - | 5 | - | 95 | 1 | 2 | 4.9 |

From Table 1 above, it was confirmed that the solvent composition according to an embodiment of the present invention had an appropriate viscosity and shear-thinning property, and that the residual ash content was very low. On the other hand, the solvent composition of the comparative examples had low viscosity, poor shear-thinning property, and high residual ash content.

To summarize the above, the configurations according to an embodiment of the present invention and variations thereof will be described below.
[1] A compound represented by Formula (1).
[2] The compound according to [1], in which R² and R³ in Formula (1) are the same or different and represent a divalent aliphatic hydrocarbon group having 2, 4, 6, or 8 carbons.
[3] The compound according to [1], in which R² and R³ in Formula (1) are the same or different and represent a linear alkylene group having 2, 4, 6, or 8 carbons.
[4] The compound according to [1], in which R² and R³ in Formula (1) are the same or different and represent a divalent aliphatic hydrocarbon group having 2 or 4 carbons.
[5] The compound according to [1], in which R² and R³ in Formula (1) are the same or different and represent a linear alkylene group having 2 or 4 carbons.
[6] The compound according to [1], in which R² and R³ in Formula (1) are the same or different and represent a divalent aliphatic hydrocarbon group having 2 carbons.
[7] The compound according to [1], in which R² and R³ in Formula (1) are the same and are a linear alkylene group having 2 carbons.
[8] The compound according to any one of [1] to [7], in which R⁴ in Formula (1) is a linear or branched alkylene group having from 1 to 8 carbon(s).
[9] The compound according to any one of [1] to [7], in which R⁴ in Formula (1) is a linear alkylene group having from 1 to 7 carbon(s).
[10] The compound according to any one of [1] to [7], in which R⁴ in Formula (1) is a linear alkylene group having from 3 to 7 carbons.
[11] The compound according to any one of [1] to [7], in which R⁴ in Formula (1) is a linear alkylene group having from 3 to 6 carbons.
[12] The compound according to any one of [1] to [7], in which R⁴ in Formula (1) is a linear alkylene group having from 3 to 5 carbons.
[13] The compound according to any one of [1] to [12], in which R⁵ and R⁶ in Formula (1) are the same or different and represent a monovalent aliphatic hydrocarbon group having from 1 to 3 carbon(s).
[14] The compound according to any one of [1] to [12], in which R⁵ and R⁶ in Formula (1) are the same or different and represent a linear or branched alkyl group having from 1 to 3 carbon(s).
[15] The compound according to any one of [1] to [12], in which R⁵ and R⁶ in Formula (1) are the same or different and represent a linear alkyl group having from 1 to 3 carbon(s).
[16] The compound according to any one of [1] to [12], in which R⁵ and R⁶ in Formula (1) are the same and are a methyl group.
[17] The compound according to any one of [1] to [16], the compound being at least one selected from the compounds represented by Formulas (1-1) to (1-9).
[18] A compound represented by Formula (2) below.
[19] The compound according to [18], the compound being at least one selected from the compounds represented by Formulas (2-1) to (2-9).
[20] A method of producing a compound represented by Formula (2), the method including reacting a compound represented by Formula (3) and a compound represented by Formula (4), or reacting a compound represented by Formula (3') and a compound represented by Formula (4') to obtain the compound represented by Formula (2).
[21] A method of producing a compound represented by Formula (1), the method including oxidizing the compound represented by Formula (2) to obtain the compound represented by Formula (1).
[22] The method of producing a compound represented by Formula (1) according to [21], in which, per 1 mol of the compound represented by Formula (2), 0.1 to 10 mol of hydrogen peroxide is reacted to oxidize the compound represented by Formula (2).
[23] A solvent composition including a miscible material of the compound described in any one of [1] to [17] and a fluid organic material.
[24] The solvent composition according to [23], in which a viscosity of the fluid organic material at 25°C and a shear rate of 1 s⁻¹ measured by a rheometer is less than 0.5 Pa·s.
[25] The solvent composition according to [23] or [24], in which the fluid organic material is at least one selected from a hydrocarbon oil, an ether, a halogenated hydrocarbon, a petroleum component, an animal and vegetable oil, a silicone oil, an ester, an aromatic carboxylic acid, pyridine, and an alcohol.
[26] The solvent composition according to any one of [23] to [25], in which a viscosity at 25°C and a shear rate of 0.3 s⁻¹ measured by a rheometer is not less than 10 Pa·s.
[27] The solvent composition according to any one of [23] to [26], in which a viscosity at 25°C and a shear rate of 0.1 s⁻¹ measured by a rheometer is not less than 10 Pa·s.
[28] The solvent composition according to any one of [23] to [27], in which a viscosity ratio, which is [(viscosity at 25°C and a shear rate of 1 s⁻¹ measured by a rheometer) / (viscosity at a shear rate of 10 s⁻¹ measured by a rheometer)] is greater than 1.5.
[29] The solvent composition according to any one of [23] to [28], in which an amount of at least one polymer compound selected from ethyl cellulose resin, alkyl cellulose resin, polyvinyl acetal resin, and acrylic resin contained therein is not greater than 10 wt.% of the total amount of the solvent composition.
[30] The solvent composition according to [29], in which the polymer compound is a polymer compound having a molecular weight of not less than 10000.
[31] The solvent composition according to any one of [23] to [28], in which an amount of a polymeric compound having a molecular weight of not less than 10000 contained therein is not greater than 10 wt.% of the total amount of the solvent composition.
[32] The solvent composition according to any one of [23] to [31], which is used for producing an electronic device.
[33] The solvent composition according to any one of [23] to [31], which is used for producing a multilayer ceramic capacitor.
[34] An ink including the solvent composition described in any one of [23] to [33].
[35] An ink for producing an electronic device including the solvent composition described in any one of [23] to [33].
[36] A conductive ink including the solvent composition described in any one of [23] to [33].
[37] A method of producing an electronic device including making a wiring and/or an electrode of the electronic device using the ink described in any one of [34] to [36].
[38] An adhesive for producing an electronic device, the adhesive including the solvent composition described in any one of [23] to [33].
[39] A method of producing an electronic device using the adhesive described in [38].
[40] A method of producing a solvent composition, the method including blending the compound described in any one of [1] to [17] and a fluid organic material to make them miscible to obtain the solvent composition.
[41] The method of producing a solvent composition according to [40], in which the method includes blending the compound described in any one of [1] to [17] at a ratio from 0.1 to 100 parts by weight to 1000 parts by weight of the fluid organic material to make them miscible.
[42] A method of producing a solvent composition, in which the method includes blending the composition represented by Formula (1) and the fluid organic material to make them miscible so as to obtain the solvent composition according to any one of [23] to [33].

### Industrial Applicability

The compound represented by Formula (1) according to an embodiment of the present invention can be suitably used as a thickening stabilizer for things such as paint, adhesives, inks, lubricating oils, pharmaceuticals, quasi drugs, and cosmetic products.

Furthermore, the solvent composition according to an embodiment of the present invention that includes the compound represented by Formula (1) has an appropriate viscosity and shear-thinning property. Therefore, the solvent composition is unlikely to cause dripping, and has good coating properties. Furthermore, the solvent composition can be calcined at low temperatures, and the residual ash content after calcining can be significantly reduced. Therefore, the solvent composition is particularly useful as a solvent for an ink for producing an electronic device or a solvent for an adhesive for producing an electronic device.

## Claims

1. A compound represented by Formula (1): 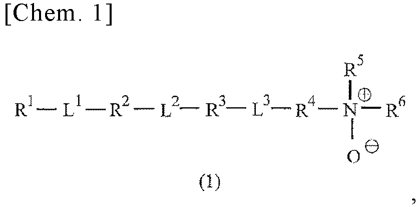 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-.

2. A compound represented by Formula (2): 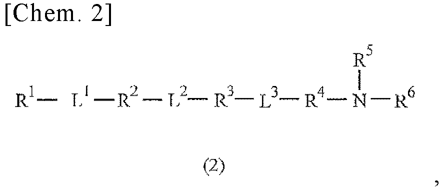 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-.

3. A method of producing a compound represented by Formula (2), the method comprising reacting a compound represented by Formula (3) and a compound represented by Formula (4), or reacting a compound represented by Formula (3') and a compound represented by Formula (4') to obtain a compound represented by Formula (2): 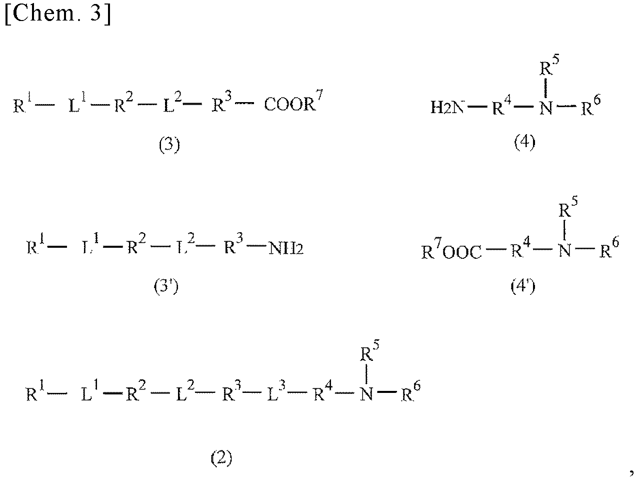 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-; R⁷ represents a hydrogen atom or a alkyl group having from 1 to 3 carbon(s); and in Formula (3), OR⁷, together with a hydrogen atom that constitutes L², may form a ring through a dehydration condensation or a dealcoholization condensation.

4. A method of producing a compound represented by Formula (1), the method comprising oxidizing a compound represented by Formula (2): 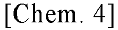 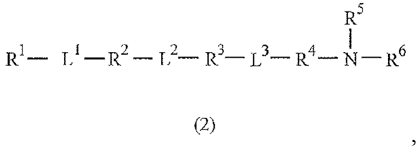 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-;
so as to obtain the compound represented by Formula (1): 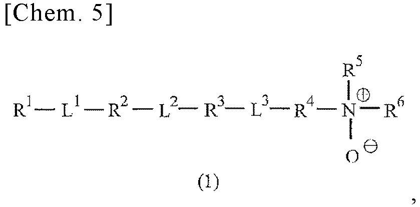 where R¹ to R⁶ and L¹ to L³ are the same as above.

5. A solvent composition for producing an electronic device, the solvent composition comprising a miscible material of the composition according to claim 1 and a fluid organic material.

6. The solvent composition for producing an electronic device according to claim 5, wherein the fluid organic material is at least one selected from a hydrocarbon oil, an ether, a halogenated hydrocarbon, a petroleum component, an animal and vegetable oil, a silicone oil, an ester, an aromatic carboxylic acid, pyridine, and an alcohol.

7. A method of producing a solvent composition for producing an electronic device, the method comprising making a compound represented by Formula (1) and a fluid organic material miscible to obtain the solvent composition for producing the electronic device according to claim 5 or 6: 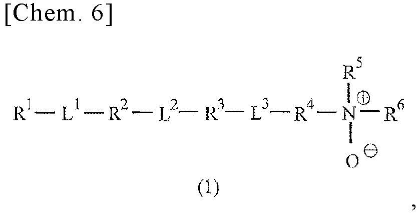 where R¹ represents a monovalent linear aliphatic hydrocarbon group having from 10 to 25 carbons; R² and R³ are the same or different and represent a divalent aliphatic hydrocarbon group having 2, 4, 6, or 8 carbons, a divalent alicyclic hydrocarbon group having 6 carbons, or a divalent aromatic hydrocarbon group; R⁴ represents a divalent aliphatic hydrocarbon group having from 1 to 8 carbon(s); R⁵ and R⁶ are the same or different and represent a monovalent aliphatic hydrocarbon group having from 1 to 3 carbon(s) or a hydroxyalkylether group; L¹ to L³ represent an amide bond; and in a case where L¹ and L³ are -CONH-, L² is -NHCO-, and in a case where L¹ and L³ are - NHCO-, L² is -CONH-.
